# EUROPÄISCHE PATENTSCHRIFT

(11) **EP 1 903 030 B1**
(45) Veröffentlichungstag und Bekanntmachung des Hinweises auf die Patenterteilung: **04.06.2014**
(21) Anmeldenummer: 07115366.2
(22) Anmeldetag: 31.08.2007
(51) Int. Cl.: C07C 253/14, C07C 255/50, C07D 213/78, C07D 213/84, C07D 213/85, C07D 239/28, C07D 209/08, C07D 307/68, C07D 277/56

(54) **Verfahren zur katalytischen Herstellung von aromatischen oder heteroaromatischen Nitrilen**
Method for catalytic manufacture of aromatic or heteroaromatic nitriles
Procédé destiné à la fabrication catalytique de nitriles aromatiques ou hétéroaromatiques

(30) Priorität: 09.09.2006 DE 102006042439
(43) Veröffentlichungstag der Anmeldung: 26.03.2008
(73) Patentinhaber: Saltigo GmbH, 51369 Leverkusen (DE)
(72) Erfinder: Müller, Nikolaus, 40789, Monheim (DE); Mägerlein, Wolfgang, 68165, Mannheim (DE); Beller, Matthias, 18211, Ostseebad Nienhagen (DE); Schareina, Thomas, 18195, Cammin (DE); Zapf, Alexsander, 83024, Rosenheim (DE)
(74) Vertreter: Pettrich, Klaus-Günter

(56) Entgegenhaltungen:
- DE-A1-102005 009 517
- DE-C- 293 094

## Beschreibung

Die vorliegende Erfindung betrifft ein Verfahren zur Herstellung von aromatischen oder heteroaromatischen Nitrilen durch Cyanierung der entsprechenden Arylhalogenide in Gegenwart von Kupfer-Katalysatoren und Gelbem Blutlaugensalz (K₄[Fe(CN)₆]) oder Rotem Blutlaugensalz (K₃[Fe(CN)₆]).

Aromatische und heteroaromatische Nitrile haben technische Bedeutung als Feinchemikalien, Agro- und Pharmazwischenprodukte. Daher sind Verfahren zu ihrer Herstellung von industrieller Bedeutung. Eine bekannte und großtechnisch angewandte Methode zur Herstellung von aromatischen Nitrilen ist die Ammonoxidation von substituierten Toluolen. Dieses Verfahren ist jedoch nur praktisch anwendbar, wenn die entsprechenden Ausgangsmaterialien (Toluole) kostengünstig verfügbar sind. Darüber hinaus gelingt die Ammonoxidation nicht in Gegenwart von oxidationsempfindlichen Substituenten im Substrat. Weitere technische Verfahren zur Darstellung von Benzonitrilen sind Umsetzungen von Carbonsäuren und Ammoniumsalzen oder Amiden durch Destillation mit stark wasserbindenden Substanzen (z.B. P₂O₅) sowie Reaktion von Carbonsäuren oder Estern in der Dampfphase mit Ammoniak über einem Al-Festbett bei 500 °C. Derartige Verfahren besitzen jedoch Nachteile aufgrund der drastischen Reaktionsbedingungen und können in der Regel nicht auf komplexer substituierte aromatische Nitrile angewandt werden.

Alternative kostengünstige Ausgangsmaterialien für aromatische Nitrile stellen die entsprechenden Arylchloride und -bromide dar. Allerdings gelingt die Substitution des Halogenids durch Cyanid nach bekannten Verfahren meist nur unbefriedigend. Beispielsweise reagieren aromatische Halogenide mit HCN in der Dampfphase bei 650°C oder bei 480-650°C in Anwesenheit eines Metall- oder Metalloxidkatalysators. Katalysatoren, die die Umsetzung von Arylhalogeniden mit Cyanid bei milderen Reaktionsbedingungen beschleunigen, sind Palladium-, Nickel- und Kupferkomplexe. So beschreiben R. Breitschuh, B. Pugin, A. Indolese und V. Gisin (EP 0 787 124 B1 und US 5,883,283) die Darstellung von substituierten 3-Aminobenzonitrilen aus den entsprechenden substituierten 3-Aminochlorbenzolen in Gegenwart von bevorzugt Ni-Komplexen und stöchiometrischen Mengen einer komplexierenden Substanz. Nachteilig bei diesem Verfahren ist die Verwendung eines Überschusses an Reduktionsmittel und die Beschränkung der Reaktion auf eine spezielle Substratklasse.

B. R. Cotter (US 4,211,721) beschreibt den positiven Einfluss von Etherkomponenten aus der Gruppe 18-Krone-6, Polyether, Alkoxypolyether oder Mixturen dieser mit einer Molmasse 200-25.000 als Co-Katalysator auf die palladiumkatalysierte Cyanierung von Arylhalogeniden.

J. B. Davison, R. J. Jasinski und P. J. Peerce-Landers (US 4,499,025) beschreiben die Herstellung aromatischer Nitrile aus Chloraromaten, katalysiert durch einen Gruppe-VIII-Metall(0)komplex, welcher elektrochemisch gebildet wird. Diese Verfahrensweise ist jedoch im Vergleich zu herkömmlichen Batch-Verfahren außerordentlich teuer.

M.-H. Rock und A. Marhold (DE 197 06 648 A1 und WO 98/37 058) beschreiben die Herstellung aromatischer Nitrile aus Chloraromaten in Gegenwart eines Nickelkatalysators und eines Ketons durch Umsetzung mit Cyaniden. Die Reaktion kann jedoch nur dann erfolgreich durchgeführt werden, wenn die Cyanidkonzentration genau kontrolliert wird, da ansonsten der Katalysator irreversibel cyaniert wird. Nachteilig bei diesem Verfahren ist wiederum der notwendige Zusatz eines Reduktionsmittels wie Zink und die Verwendung von speziellen Ketonen als Lösemittel.

M. Beller und Mitarbeiter beschreiben den Einfluss von Kronenethern, Diphosphinliganden und Diaminliganden auf die palladiumkatalysierte Umsetzung von Arylhalogeniden mit Alkalicyaniden (DE 101 13 976, Tetrahedron Lett. 2001, 42, 6707-10). Auf diesen Arbeiten fußend, wurden Verfahren entwickelt, die auf einer dosierten Zugabe von Acetoncyanhydrin (Angew. Chem. 2003, 115, 1700-3), Trimethylsilylcyanid (J. Organomet. Chem. 2003, 684, 50-5) oder Blausäure (DE 103 22 408.4) als Cyaniddonor beruhen. Nachteilig ist hier die Verwendung von teuren Palladiumkatalysatoren und speziellen Liganden.

A. Viauvy und M. Casado (EP 0 994 099 A1) beschreiben weiterhin die Umsetzung von Chloraromaten zum entsprechenden Nitril mit stöchiometrischen Mengen Kupfercyanid und einer Bromidquelle oder Alkalimetallcyanid bzw. Tetraalkylammoniumcyanid in Anwesenheit von Kupferbromid und einem Phasentransferkatalysator oder Kupfercyanid und Lithiumiodid. Nachteilig ist hier der Einsatz stöchiometrischer Mengen des Übergangsmetalls.

Eine kupferkatalysierte Cyanierung von Arylhalogeniden wurde von Wu et al. beschrieben (Tetrahedron Lett. 2002, 43, 387-389). Sie verwenden 5 Mol-% eines Kupfer(I)salzes als Katalysator und Natriumcyanid als Cyanidquelle. Gute Ausbeuten können allerdings nur bei der Umsetzung reaktiver und teurer lodaromaten erzielt werden. Ein weiterer Nachteil dieses Verfahrens ist der Einsatz von ionischen Flüssigkeiten als Lösungsmittel, die teuer und nur aufwendig zu reinigen sind.

J. Zanon, A. Klapars und S. L. Buchwald (J. Am. Chem. Soc. 2003, 125, 2890-1) beschreiben die Cyanierung von Bromaromaten mit Natriumcyanid in Gegenwart von 10 Mol-% Kupfer(I)iodid als Katalysator und 20 mol% Kaliumiodid als Co-Katalysator. Weiterhin wird ein Äquivalent N,N'-Dimethylethylendiamin zugegeben. Es wird angenommen, dass die Arylbromide intermediär in die entsprechenden -iodide überführt werden, die anschließend cyaniert werden.

In einer ebenfalls mit Kupfer(I)lodid katalysierten Methode von Cristau et al. (Chem. Eur. J. 2005, 11, 2483-2492) werden 20 Mol-% des Liganden 1,10-Phenanthrolin und Acetoncyanhydrin als Cyaniddonor verwendet. Auch hier muss Kaliumiodid als Co-Katalysator zugesetzt werden.

Ein wesentlicher Nachteil aller bisher beschriebenen katalytischen Cyanierungen ist die z.T. extrem hohe Toxizität der verwendeten Cyanierungsmittel, die darauf beruht, dass bei Kontakt mit Wasser Blausäure freigesetzt wird. Von M. Beller et al. wurden erstmals katalytische Cyanierungen mit dem nicht toxischen Gelben Blutlaugensalz [Kaliumhexacyanoferrat(II), Chem. Commun. 2004, 1388-1389] beschrieben. Nachteilig bei diesem Verfahren ist jedoch, dass die Umsetzungen nur in Verbindung mit einem Palladiumkatalysator gelingen.

Weiterhin beschreiben T. Schareina, A. Zapf und M. Beller (Tetrahedron Lett. 2005, 46, 2585-2588) die Cyanierung von Arylbromiden mit Cu-Katalysatoren in Gegenwart des Liganden N,N'-Dimethylethylendiamin. Aufgrund des hohen Preises dieses Liganden sind technische Anwendungen unrealistisch.

Die DE 10 2005 009517 beschreibt eine kupferkatalysierte Cyanierung von (Hetero)aromatischen Arylhalogeniden in Gegenwart von Kaliumhexacyanoferrat (II) als Cyanidquelle. Aliphatische und araliphatische Amine wie Triethylamin und N,N-Dimethylaniline oder Alkylamine mit mindestens 2 Stickstoffatomen werden dabei als Additive eingesetzt.

Die DE 293094 beschreibt die Zugabe von Pyridin als reaktionsförderndes Mittel in der katalytischen Cyanierung von Arylhalogeniden.

Zusammenfassend bleibt festzuhalten, dass fast alle bisher bekannten Übergangsmetallkatalysierten Cyanierungen von Arylhalogeniden entweder toxische Cyanidquellen und/oder teure Katalysatorsysteme verwenden.

Aufgabe der vorliegenden Erfindung war daher die Entwicklung eines verbesserten Verfahrens zur Cyanierung von Halogenaromaten. Insbesondere sollte dieses Verfahren im technischem Maßstab gut einsetzbar sein und den Verfahren des Standes der Technik bezüglich der Katalysatorkosten und der Toxizität der Cyanidquelle überlegen sein. Überraschenderweise wurde gefunden, dass eine Kombination von Kupferverbindungen und kostengünstigen Additiven die Reaktion von Arylhalogeniden mit ungiftigen Cyanidquellen, wie zum Beispiel Kaliumhexacyanoferrat(II) oder Kaliumhexacyanoferrat(III) (gelbem bzw. rotes Blutlaugensalz) katalysiert.

Die gestellte Aufgabe wurde anspruchsgemäß gelöst, dadurch, dass man in einem Verfahren zur katalytischen Herstellung von gegebenenfalls substituierten aromatischen oder hetereoaromatischen Nitrilen der allgemeinen Formel (I)

Ar-CN (I)

die Umsetzung der entsprechenden Arylhalogenide der allgemeinen Formel (II),

Ar-X (II)

worin X für Chlor, Brom, Iod oder Sulfonat, bevorzugt für Chlor und Brom, besonders bevorzugt für Brom, und Ar für einen gegebenenfalls substituierten aromatischen oder heteroaromatischen Rest steht,
unter Verwendung von Kaliumhexacyanoferrat(II) oder Kaliumhexacyanoferrat(III) als Cyaniddonor in Gegenwart von Kupferverbindungen und 1-Alkylimidazolen durchführt.

Das erfindungsgemäß verwendete Cyanierungsreagenz Kaliumhexacyanoferrat(II) ist ungiftig, löst sich in Wasser ohne Zersetzung und wird sogar in der Lebensmittelindustrie z.B. bei der Herstellung von Tafelsalz oder zum Schönen von Weinen eingesetzt (Roempp Lexikon Chemie, Georg Thieme Verlag, Stuttgart/New York, 1999).

Als Kupferverbindungen können bekannte Kupfer(I)- und Kupfer(II)-Verbindungen eingesetzt werden. Typische Beispiele sind die Kupferhalogenide wie CuI, CuBr, Kupfercarboxylate wie Cu(OAc)₂, Kupfercyanide wie CuCN, Kupferalkoxide wie Cu(acac)₂, Kupferaqua- und Kupferammin-Komplexe wie [Cu(NH₃)₄]SO₄, aber auch kationische Kupfer-Verbindungen wie Cu(BF₄)₂. Bevorzugt sind Kupferhalogenide und Kupfer(II)-tetrafluoroborat.

Die eingesetzte Kupferverbindung sollte in einer ausreichenden Menge im Reaktionsgemisch vorhanden sein. Der Fachmann wird die notwendige Einsatzmenge anhand von Wirtschaftlichkeitserwägungen (Schnelligkeit der Reaktion, Ausbeute, Stoffeinsatzkosten) auswählen. Beim erfindungsgemäßen Verfahren können Turnover-Werte der Katalysatoren in der Größenordnung von mindestens 10 bis 100.000 realisiert werden. Bevorzugt ist der Einsatz der Kupferverbindung in einer Menge von 100 ppm bis 100 Mol-% bezogen auf das eingesetzte Arylhalogenid. Besonders bevorzugt werden 1 mol% bis 30 mol% eingesetzt.

Als Lösungsmittel finden bei dem erfindungsgemäßen Verfahren im allgemeinen inerte organische Lösungsmittel und/oder Wasser Verwendung. Als vorteilhaft haben sich dipolar aprotische Lösungsmittel wie z.B. aliphatische Ester oder Amide, heteroaromatische Lösungsmittel wie 1-substituierte Imidazole sowie deren Gemische, insbesondere mit Toluol und Xylol, erwiesen. Besonders vorteilhaft ist die Verwendung von 1-Alkylimidazolen wie 1-Methyl- und 1-Butylimidazol.

Die Reaktion wird bei Temperaturen von 20 bis 220°C durchgeführt. Insbesondere wird bei Reaktionstemperaturen von 80 bis 200°C, besonders bevorzugt bei 100 bis 180°C, gearbeitet.

Die Reaktion wird normalerweise drucklos durchgeführt. Sie kann jedoch auch unter Druck, z.B. in einem Autoklaven oder Druckrohr, durchgeführt werden.

Es werden reaktionsbeschleunigende bzw. katalysatorstabilisierende Additive zugesetzt. Als Additive werden dabei 1-Alkyl-substituierte Imidazole wie 1-Methylimidazol, 1-Ethylimidazol, 1-Propylimidazol, 1-Isopropylimidazol, 1-Butylimidazol, 1-sec. Butylimidazol, 1-tert. Butylimidazol, 1-Octylimidazol, 1-Benzylimidazol etc., 1-Methylbenzimidazol, 1-Ethylbenzimidazol, 1-Propylbenzimidazol, 1-Isopropylbenzimidazol, 1-Butylbenzmidazol, 1-sec. Butylbenzimidazol, 1-Octylbenzimidazol, 1-Benzylbenzimidazol etc. eingesetzt. Bevorzugt ist die Verwendung von 1-Methylimidazol und 1-Butylimidazol.

Die Imidazole werden im allgemeinen im Verhältnis 1:1 bis 10000:1 (Molverhältnis. Ligand:Katalysator) zum Katalysator eingesetzt. Gegebenenfalls dienen sie als Lösungsmittel.

Unter Umständen führt ein Zusatz mehrer Liganden zu synergistischen positiven Effekten.

Mit den beim erfindungsgemäßen Verfahren eingesetzten Cyanidquellen Kaliumhexacyanoferrat(II) oder Kaliumhexacyanoferrat(III) und dem entsprechenden Katalysatorsystem aus einer Kombination einer Kupferverbindung und einem 1-Alkylimidazol lassen sich bei der vorliegenden Umsetzung signifikant bessere Ergebnisse realisieren als mit allgemein bekannten Reaktionssystemen. Gegenüber dem Stand der Technik ist als deutlicher Fortschritt zu sehen: 1. der Einsatz kostengünstiger Kupfer- statt teurer Palladium-Katalysatoren, 2. die Verwendung einer ungiftigen und ungefährlichen Cyanidquelle, die unter wesentlich geringeren Sicherheitsvorkehrungen gehandhabt werden kann als herkömmliche Cyanidquellen, 3. der Wegfall von Iodidzusätzen, 4. eine wesentlich größere Substratbreite und 5. die Verwendung signifikant kostengünstigerer und technisch sowie kommerziell leicht verfügbarer Additive anstelle teurer Ligandsysteme. So zeigt ein Vergleich verschiedener Cyanierungsverfahren (Tabelle 3) für verschiedene Substrate deutliche Vorteile des hier beschriebenen erfindungsgemäßen Verfahrens gegenüber bekannten state-of the-art-Verfahren.

Grundsätzlich besteht hinsichtlich des Einsatzes von Aromaten oder Heteroaromaten keine Beschränkung. Insbesondere kann der Rest Ar ein (C₆-C₁₉)-Arylrest bzw. ein (C₃-C₁₈)-Heteroarylrest mit 1, 2 oder 3 Heteroatome wie z.B. Stickstoff, Sauerstoff oder Schwefel im Ring sein.

Dabei ist es möglich, dass der Rest Ar bis zu acht Substituenten tragen kann, die unabhängig voneinander (C₁-C₈)-Alkyl, (C₃-C₈)-Cycloalkyl, (C₂-C₈)-Alkenyl, (C₂-C₈)-Alkinyl, (C₇-C₂₀)-Aralkylrest, OH, O-1(C₁-C₈)-Alkyl], OC(O)-1(C₁-C₈)-Alkyl], O-Phenyl, Phenyl, NH₂, NO₂, NO, N[(C₁-C₈)-Alkyl]₂, NH[(C₁-C₈)-Alkyl], NHC(O)-1(C₁-C₈)-Alkyl], N[(C₁C₈)-A)kyl]C(O)-[(C₁C₈)-Alkyl], SH, S-Phenyl, S-[(C₁-C₈)-Alkyl], Fluor, Chlor, CF₃, CN, COOH, COO-[(C₁-C₈)-Alkyl], CONH-[(C₁-C₈)-Alkyl], COO-Phenyl, CONH-Phenyl, CHO, SO₂(C₁-C₈)-Alkyl, SO-(C₁-C₈)-Alkyl, PO-(Phenyl)₂, PO-[(C₁-C₈)-Alkyl]₂, PO₃H₂, PO[O-(C₁-C₈)-Alkyl]₂, SO₃H, SO₃M, SO₃-[(C₁-C₈)-Alkyl], Si[(C₁-C₈)-Alkyl]₃, (C₁-C₈)-Haloalkyl sowie (C₁-C₈)-Acyl sein können.

Als (C₁-C₈)-Alkyl sind anzusehen Methyl, Ethyl, n-Propyl, Isopropyl, n-Butyl, Isobutyl, sec-Butyl, tert-Butyl, Pentyl, Hexyl, Heptyl oder Octyl samt aller Bindungsisomeren. Diese können einfach oder mehrfach mit (C₁-C₈)-Alkoxy, (C₁-C₈)-Haloalkyl, OH, Halogen, NH₂, NO₂, SH, S-(C₁C₈)-Alkyl substituiert sein.

Als (C₂-C₈)-Alkenyl ist mit Ausnahme von Methyl ein wie oben dargestellter (C₁-C₈)-Alkyl-Rest zu verstehen, der mindestens eine Doppelbindung aufweist.

Unter (C₂-C₈)-Alkinyl ist mit Ausnahme von Methyl ein wie oben dargestellter (C₁-C₈)-Alkyl-Rest zu verstehen, der mindestens eine Dreifachbindung aufweist.

Unter (C₁-C₈)-Acyl versteht man einen über eine -C=O-Funktion ans Molekül gebundenen (C₁-C₈)-Alkyl-Rest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltigen Resten substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₆-C₁₉)-Arylrest wird ein aromatischer Rest mit 6 bis 19 C-Atomen verstanden. Insbesondere zählen hierzu Verbindungen wie Phenyl-, Naphthyl-, Anthryl-, Phenanthryl-, Biphenylreste. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Ein (C₇-C₂₀)-Aralkylrest ist ein über einen (C₁-C₈)-Alkylrest an das Molekül gebundener (C₆-C₁₉)-Arylrest.

(C₁-C₈)-Alkoxy ist ein über ein Sauerstoffatom an das betrachtete Molekül gebundener (C₁-C₈)-Alkyl-Rest.

(C₁-C₈)-Haloalkyl ist ein mit einem oder mehreren Halogenatomen substituierter (C₁-C₈)-AlkylRest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches 1, 2 oder 3 Heteroatome wie z.B. Stickstoff, Sauerstoff oder Schwefel im Ring aufweist. Als solche Heteroaromaten werden insbesondere Reste angesehen wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl. Unter einem (C₄-C₁₉)-Heteroaralkyl wird ein dem (C₇-C₂₀)-Aralkylrest entsprechendes heteroaromatisches System verstanden.

Als Halogene kommen Fluor, Chlor, Brom und Iod in Frage.

### Beispiele:

### Allgemeine Arbeitsvorschrift:

In einem Autoklaven wurden 1 Äquiv. Aryl- oder Heteroarylhalogenid, 2 Äquiv. 1-Alkylimidazol, 0.1 Äquiv. CuI, 0.2 Äquiv. getrocknetes K₄[Fe(CN)₆] (Gelbes Blutlaugensalz), Tetradekan als interner Standard für die GC-Analytik und eine geeignete Menge Toluol unter Argon zusammengegeben und auf 160°C erhitzt. (Die Trocknung des K₄[Fe(CN)₆] erfolgte durch Erwärmen von gepulvertem K₄[Fe(CN)₆]×3H₂O in einem Vakuum von ≤ 1 mbar auf 80°C für mindestens 24 Stunden.) Nach 16 Stunden wurde die Reaktionsmischung auf Raumtemperatur abgekühlt. Umsatz und Ausbeute konnten über Gaschromatographie bestimmt werden. Eine Isolierung des Produktes fand gemäß den üblichen Aufarbeitungen (Destillation, Kristallisation oder Chromatographie) statt.

**Tabelle 1: Kupferkatalysierte Cyanierung mit Kaliumhexacyanoferrat(II) in Gegenwart von 1-Alkylimidazolen.**

| **Beispiel Nr.** | **Ar-X** | **Ausbeute an Produktnitril [%] (Additiv)** |
|---|---|---|
| 1. | 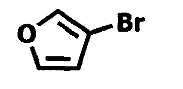 3-Bromfuran | 49 (1-Butylimidazol) |
| 2. | 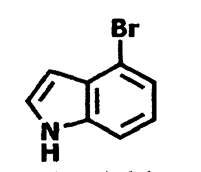 4-Bromindol | 50 (1-Methylimidazol) |
| 3. | 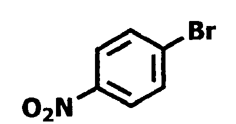 1-Brom-4-nitrobenzol | 80 (1-Butylimidazol) |
| 4. | 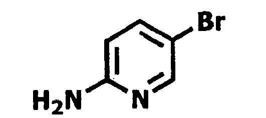 2-Amino-5-brompyridin | 55 (1-Methylimidazol) |
| 5. | 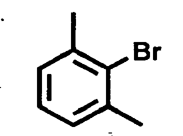 2-Brom-m-xylol | 66 (180°C); (1-Methylimidazol) |
| 6. | 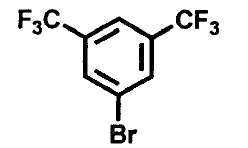 3,5-Bis(trifluormethyl)-brombenzol | 98 (1-Butylimidazol) 68 (1-Methylimidazol, 0,05 Äquiv. CuI) 52 (1-Butylimidazol, 0,02 Äquiv. CuI) 29 (1-Butylimidazol, 0,02 Äquiv. CuBr) |
| 7. | 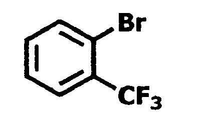 2-Brombenzotrifluorid | 83 (1-Butylimidazol) |
| 8. | 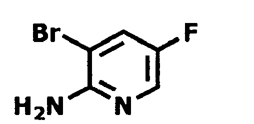 2-Amino-3-brom-5-fluorpyridin | 93 (1-Methylimidazol) |
| 9. | 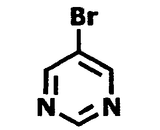 5-Brompyrimidin | 95 (1-Butylimidazol) 82 (1-Methylimidazol, 0,05 Äquiv. CuI) |
| 10. | 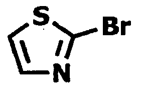 2-Bromthiazol | 99 (1-Methylimidazol) |
| 11. | 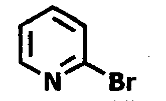 2-Brompyridin | 100 (1-Methylimidazol) |

**Tabelle 2: Vergleich verschiedener Cyanierungsreagentien bei der Cyanierung von 3,5-Bis(trifluormethyl)-brombenzol mit 200 mol% 1-Butylimidazol.**

| **Beispiel Nr.** | **Äquiv. Cyanierungsreagens** | **Ausbeute an 3,5-Bis(trifluormethyl) -benzonitril [%]** |
|---|---|---|
| 12. | 0.2 K₄[Fe(CN)₆] (140 °C) | 77 |
| 13. | 0.2 K₃[Fe(CN)₆] (120 °C) | 60 |
| 14. | 1.1 KCN (120 °C) | 66 |

**Tabelle 3: Vergleich verschiedener Cyanierungsmethoden.**

| **Substrat** | **Lösungsmittel** | **Temperatur [°C]** | **Metallvorstufe¹** | **Additive¹** | **Ligand¹** | **Ausbeute [%]** |
|---|---|---|---|---|---|---|
| 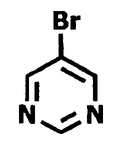 5-Brom-pyrimidin | NMP | 140 | Pd(OAc)₂ 0,1% | Na₂CO₃ 20% | dppf²0,2% | 0 |
| | NMP | 140 | CuI 10% | KI 20% | DMEDA 100% | 0 |
| | Toluol | 160 | CuI 10% | - | 1-Butyl-imidazol 200% | 95 |

| | | | | | | |
|---|---|---|---|---|---|---|
| ¹ Alle Prozentangaben beziehen sich auf das Substrat. ² dppf = 1,1'-Bis-(diphenylphosphino)-ferrocen. | | | | | | |

| **Substrat** | **Lösungsmittel** | **Temperatur [°C]** | **Metallvorstufe¹** | **Additive¹** | **Ligand¹** | **Ausbeute [%]** |
|---|---|---|---|---|---|---|
| 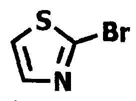 2-Bromthiazol | NMP | 140 | CuI 10% | Na₂CO₃ 20% KI20% | DMEDA 100% | 0 |
| | NMP | 140 | Pd(OAc)20,1% | Na₂CO₃ 20% | dppf 0,2% | 0 |
| | 1-Methyl-imidazol | 140 | CuI 10% | - | - | 99 |
| 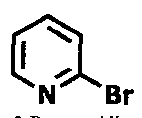 2-Brompyridin | NMP | 130 | Pd(OAc)₂ 0,5% | Na₂CO₃ 20% | dppp³ 2% | 30 |
| | NMP | 110 | Pd(OAc)₂ 0,5% | Na₂CO₃ 20% | dppf 1% | 0 |
| | NMP | 140 | Pd(OAc)₂ 0,5% | Na₂CO₃ 20% | dppf 1% | < 5 |
| | NMP | 160 | Cu(BF₄)₂*6H₂O 10% | Na₂CO₃ 20% KI 20% | DMEDA 100% | 0 |
| | 1-Methyl-imidazol | 140 | CuI 10% | - | - | 100 |

| | | | | | | |
|---|---|---|---|---|---|---|
| ³ dppp = 1,3-Bis-(diphenylphosphino)-propan. | | | | | | |

## Patentansprüche

1. Verfahren zur katalytischen Herstellung von gegebenenfalls substituierten aromatischen oder hetereoaromatischen Nitrilen der allgemeinen Formel (I)
Ar-CN (I)
durch Umsetzung der entsprechenden Arylhalogenide der allgemeinen Formel (II),
Ar-X (II)
worin X für Chlor, Brom, Iod oder Sulfonat und Ar für einen gegebenenfalls substituierten aromatischen oder heteroaromatischen Rest steht,
**dadurch gekennzeichnet, dass** die Umsetzung in Gegenwart von Kupferverbindungen und von 1-Alkylimidazolen durchgeführt wird und als Cyaniddonor Kaliumhexacyanoferrat (II) oder Kaliumhexacyanoferrat(III) eingesetzt wird.

2. Verfahren nach Anspruch 1, **dadurch gekennzeichnet, dass** als Kupferverbindungen bekannte Kupfer(I)- und Kupfer(II)-Salze oder -Komplexe eingesetzt werden.

3. Verfahren nach Anspruch 1 und 2, **dadurch gekennzeichnet, dass** man als Kupferverbindungen Kupferhalogenide, Kupfercyanide oder Kupfer(II)-tetrafluoroborat einsetzt.

4. Verfahren nach einem der Ansprüche 1 bis 3, **dadurch gekennzeichnet, dass** als 1-Alkylimidazole 1-Methylimidazol oder 1-Butylimidazol eingesetzt wird..

5. Verfahren nach einem der Ansprüche 1 bis 4, **dadurch gekennzeichnet, dass** man die Kupferverbindung in einer Menge von 1 ppm bis 100 mol-%, bevorzugt 10 ppm bis 30 mol% besonders bevorzugt 0.1 mol% bis 10 mol%, bezogen auf das eingesetzte Arylhalogenid Ar-X, einsetzt.

6. Verfahren nach einem der Ansprüche 1 bis 5, **dadurch gekennzeichnet, dass** das Amin in einer solchen Menge eingesetzt wird, dass das molare Verhältnis Kupferverbindung zu Amin 1:1 bis 1:10000, bevorzugt 1:1 bis 1:1000 und besonders bevorzugt 1:1 bis 1:200 beträgt.

7. Verfahren nach einem der Ansprüche 1 bis 6, **dadurch gekennzeichnet, dass** die Umsetzung in einem inerten organischen Lösungsmittel und/oder Wasser erfolgt.

8. Verfahren nach einem der Ansprüche 1 bis 7, **dadurch gekennzeichnet, dass** die Umsetzung bei Temperaturen von 20 bis 220 °C, bevorzugt bei 80 bis 200 °C, besonders bevorzugt bei 100 bis 180 °C durchgeführt wird.

9. Verfahren nach einem der Ansprüche 1 bis 8, **dadurch gekennzeichnet, dass** Ar ein (C₆-C₁₉)-Arylrest bzw. ein (C₃-C₁₈)-Heteroarylrest mit 1, 2 oder 3 Heteroatomen ausgewählt aus Stickstoff, Sauerstoff oder Schwefel im Ring ist.

10. Verfahren nach einem der Ansprüche 1 bis 9, **dadurch gekennzeichnet, dass** als Arylhalogenid 2-Brombenzotrifluorid, 3,5-Bis(trifluormethyl)brombenzol, 4-bromnitrobenzol, 2-Brom-6-methylanilin, 2-Amino-5-brom-3-methylbenzoesäure, 2-Amino-5-chlor-3-methylbenzoesäure, 5-Brompyrimidin, 2-Brom-m-Xylol, 2-Brompyridin, 2-Chlorpyridin, 3-Brompyridin, 3-Chlorpyridin, 2-Bromthiazol oder 3-Bromfuran eingesetzt wird.

## Claims

1. Process for catalytically preparing optionally substituted aromatic or heteroaromatic nitriles of the general formula (I)
Ar-CN (I)
by reacting the corresponding aryl halides of the general formula (II)
Ar-X (II)
in which X is chlorine, bromine, iodine or sulphonate and Ar is an optionally substituted aromatic or heteroaromatic radical,
**characterized in that** the reaction is performed in the presence of copper compounds and of 1-alkylimidazoles, and potassium hexacyanoferrate(II) or potassium hexacyanoferrate(III) is used as cyanide donor.

2. Process according to Claim 1, **characterized in that** the copper compounds used are known copper(I) and copper(II) salts or complexes.

3. Process according to Claims 1 and 2, **characterized in that** the copper compounds used are copper halides, copper cyanides or copper(II) tetrafluoroborate.

4. Process according to any one of Claims 1 to 3, **characterized in that** the 1-alkylimidazole used is 1-methylimidazole or 1-butylimidazole.

5. Process according to any one of Claims 1 to 4, **characterized in that** the copper compound is used in an amount of 1 ppm to 100 mol%, preferably 10 ppm to 30 mol%, more preferably 0.1 mol% to 10 mol%, based on the aryl halide (Ar-X) used.

6. Process according to any one of Claims 1 to 5, **characterized in that** the amine is used in such an amount that the molar ratio of copper compound to amine is 1:1 to 1:10 000, preferably 1:1 to 1:1000 and more preferably 1:1 to 1:200.

7. Process according to any one of Claims 1 to 6, **characterized in that** the reaction is effected in an inert organic solvent and/or water.

8. Process according to any one of Claims 1 to 7, **characterized in that** the reaction is performed at temperatures of 20 to 220°C, preferably at 80 to 200°C, more preferably at 100 to 180°C.

9. Process according to any one of Claims 1 to 8, **characterized in that** Ar is a (C₆-C₁₉) -aryl radical or a (C₃-C₁₈) -heteroaryl radical having 1, 2 or 3 heteroatoms selected from the group nitrogen, oxygen and sulphur in the ring.

10. Process according to any one of Claims 1 to 9, **characterized in that** the aryl halide used is 2-bromobenzotrifluoride, 3,5-bis(trifluoromethyl)bromobenzene, 4-bromonitrobenzene, 2-bromo-6-methylaniline, 2-amino-5-bromo-3-methylbenzoic acid, 2-amino-5-chloro-3-methylbenzoic acid, 5-bromopyrimidine, 2-bromo-m-xylene, 2-bromopyridine, 2-chloropyridine, 3-bromopyridine, 3-chloropyridine, 2-bromothiazole or 3-bromofuran.

## Revendications

1. Procédé pour la préparation catalytique de nitriles aromatiques ou hétéroaromatiques éventuellement substitués de formule générale (I)
Ar-CN (I)
par mise en réaction des halogénures d'aryle correspondants de formule générale (II),
Ar-X (II)
formules dans lesquelles X représente le chlore, le brome, l'iode ou le sulfonate et Ar représente un radical aromatique ou hétéroaromatique éventuellement substitué,
**caractérisé en ce qu'**on effectue la réaction en présence de composés contenant du cuivre et de 1-alkylimidazoles et on utilise comme donneur de cyanure l'hexacyanoferrate(II) de potassium ou l'hexacyanoferrate(III) de potassium.

2. Procédé selon la revendication 1, **caractérisé en ce qu'**on utilise comme composés contenant du cuivre des sels ou complexes de cuivre(I) et de cuivre(II) connus.

3. Procédé selon la revendication 1 et la revendication 2, **caractérisé en ce qu'**on utilise comme composés contenant du cuivre des halogénures de cuivre, des cyanures de cuivre ou le tétrafluoroborate de cuivre(II).

4. Procédé selon l'une quelconque des revendications 1 à 3, **caractérisé en ce qu'**on utilise comme 1-alkylimidazoles le 1-méthylimidazole ou le 1-butylimidazole.

5. Procédé selon l'une quelconque des revendications 1 à 4, **caractérisé en ce qu'**on utilise le composé contenant du cuivre en une quantité de 1 ppm à 100 % en moles, de préférence de 10 ppm à 30 % en moles, de façon particulièrement préférée de 0,1 % en moles à 10 % en moles, par rapport à l'halogénure d'aryle Ar-X utilisé.

6. Procédé selon l'une quelconque des revendications 1 à 5, **caractérisé en ce qu'**on utilise l'amine en une quantité telle que le rapport molaire composé contenant du cuivre à amine vaut de 1:1 à 1:10000, de préférence de 1:1 à 1:1000 et de façon particulièrement préférée de 1:1 à 1:200.

7. Procédé selon l'une quelconque des revendications 1 à 6, **caractérisé en ce que** la réaction s'effectue dans un solvant organique inerte et/ou l'eau.

8. Procédé selon l'une quelconque des revendications 1 à 7, **caractérisé en ce qu'**on effectue la réaction à des températures de 20 à 220 °C, de préférence de 80 à 200 °C, de façon particulièrement préférée de 100 à 180 °C.

9. Procédé selon l'une quelconque des revendications 1 à 8, **caractérisé en ce que** Ar est un radical aryle en C₆-C₁₉ ou un radical hétéroaryle en C₃-C₁₈ comportant dans le cycle 1, 2 ou 3 hétéroatomes choisis parmi les atomes d'azote, d'oxygène et de soufre.

10. Procédé selon l'une quelconque des revendications 1 à 9, **caractérisé en ce qu'**on utilise comme halogénure d'aryle le trifluorure de 2-bromo-benzène, le 3,5-bis(trifluorométhyl)bromobenzène, le 4-bromonitrobenzène, la 2-bromo-6-méthylaniline, l'acide 2-amino-5-bromo-3-méthylbenzoïque, l'acide 2-amino-5-chloro-3-méthylbenzoïque, la 5-bromo-pyrimidine, le 2-bromo-m-xylène, la 2-bromopyridine, la 2-chloropyridine, la 3-bromopyridine, la 3-chloropyridine, le 2-bromothiazole ou le 3-bromofurane.
